(19) Europäisches Patentamt
European Patent Office
Office européen des brevets

(11) EP 4 166 580 A1

(12) EUROPEAN PATENT APPLICATION
published in accordance with Art. 153(4) EPC

(43) Date of publication:
**19.04.2023 Bulletin 2023/16**

(21) Application number: **21822057.2**

(22) Date of filing: **04.06.2021**

(51) International Patent Classification (IPC):
***C08F 20/28*** *(2006.01)* ***C08F 20/58*** *(2006.01)*
***C09K 3/18*** *(2006.01)* ***C07C 69/653*** *(2006.01)*

(52) Cooperative Patent Classification (CPC):
**C07C 69/653; C08F 20/28; C08F 20/58; C09K 3/18**

(86) International application number:
**PCT/JP2021/021425**

(87) International publication number:
**WO 2021/251302 (16.12.2021 Gazette 2021/50)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.06.2020 JP 2020100920**

(71) Applicant: **Daikin Industries, Ltd.**
**Osaka-shi, Osaka 530-8323 (JP)**

(72) Inventors:
- **INAMASU, Rena**
  **Osaka-shi, Osaka 530-8323 (JP)**
- **HIGASHI, Masahiro**
  **Osaka-shi, Osaka 530-8323 (JP)**
- **YAMAMOTO, Ikuo**
  **Osaka-shi, Osaka 530-8323 (JP)**
- **YOSHIDA, Tomohiro**
  **Osaka-shi, Osaka 530-8323 (JP)**
- **TAKAKUWA, Tatsuya**
  **Osaka-shi, Osaka 530-8323 (JP)**
- **MATUBAYASI, Nobuyuki**
  **Suita-shi, Osaka 565-0871 (JP)**
- **KOJIMA, Hidekazu**
  **Suita-shi, Osaka 565-0871 (JP)**

(74) Representative: **Hoffmann Eitle**
**Patent- und Rechtsanwälte PartmbB**
**Arabellastraße 30**
**81925 München (DE)**

# (54) FLUORINE-CONTAINING COMPOUND

(57) There is provided a fluorine-containing compound by which high water-repellency can be obtained despite the small number of fluorine atoms. A compound represented by a general formula (1):

$$L\text{-}(R^1\text{-}X\text{-}Rf\text{-}Y\text{-}R^2)_n \ldots \qquad (1)$$

wherein L represents an n-valent organic group; n represents an integer of 1 or more; Rf represents a linear or branched fluoroalkylene group containing 1 to 4 of at least any of a $CF_2$ unit, a CFH unit and a CFRf' unit, and optionally having an ether bond, wherein Rf' represents a fluoroalkyl group having 1 carbon atom; $R^1$ represents a direct bond, or a linear or branched hydrocarbon group having 1 to 4 carbon atoms; $R^2$ represents a linear or branched hydrocarbon group having 7 to 29 carbon atoms, containing no fluorine atoms, and optionally having an ether bond; and X and Y each independently represent a direct bond or a divalent linking group.

EP 4 166 580 A1

## Description

### Technical Field

This disclosure relates to a fluorine-containing compound, as well as a polymer, an article, a composition and a textile product containing the fluorine-containing compound.

### Background Art

It has been known that a fluorine-containing compound can be used for a water- and oil-repellent agent.

For example, Patent Literature 1 discloses a water- and oil-repellent agent containing a copolymer composed of an acrylic fluoroalkyl group-containing monomer and a hydrophilic group-containing monomer, as a main ingredient, wherein the fluoroalkyl group-containing monomer is a fluorine-containing compound represented by the following general formula:

$$Rf'-R^{1'}-X'-R^{2'}C=CH_2$$

wherein, Rf' represents a linear fluoroalkyl group having 3 to 20 carbon atoms, $R^{1'}$ represents a linear or branched alkylene group having 1 to 10 carbon atoms, $R^{2'}$ represents a hydrogen atom or a methyl group, X' represents -O-C(=O)- or $-SO_2-N(A^1)-A^2-$ ($A^1$ represents an alkyl group having 1 to 4 carbon atoms, $A^2$ represents a direct bond or $-A^3-O-C(=O)-$ ($A^3$ represents an alkylene group having 1 to 4 carbon atoms)).

Specifically, Patent Literature 1 describes that Rf' represents a fluoroalkyl group containing 6 or more $CF_2$ units (the $CF_2$ unit at an end is $CF_3$).

### Citation List

#### Patent Literature

Patent Literature 1: JP-A-2000-290640

#### Non Patent Literature

Non Patent Literature 1: S. Sakuraba et al., "ERmod: Fast and Versatile Computation Software for Solvation Free Energy with Approximate Theory of Solutions", Journal of Computational Chemistry, 2014, Volume 35, Issue 21, pp. 1592-1608

### Summary of Invention

#### Technical Problem

Products produced using fluorine-containing compounds have been required to have a fluorine content as low as possible in recent years. However, it is generally considered that lowering the number of fluorine atoms in a fluorine-containing compound lowers water-repellency brought about by the compound.

An object of the disclosure is to provide a fluorine-containing compound by which high water-repellency can be obtained despite the small number of fluorine atoms. Another object of the disclosure is to provide a polymer, an article, a composition and a textile product containing such a fluorine-containing compound.

#### Solution to Problem

This disclosure encompasses the following embodiments.

[1] A compound represented by a general formula (1):

$$L-(R^1-X-Rf-Y-R^2)_n \quad ... \qquad (1)$$

wherein L represents an n-valent organic group,
n represents an integer of 1 or more,

Rf represents a linear or branched fluoroalkylene group containing 1 to 4 of at least any of a $CF_2$ unit, a CFH unit and a CFRf' unit, and optionally having an ether bond, wherein Rf' represents a fluoroalkyl group having 1 carbon atom,

$R^1$ represents a direct bond, or a linear or branched hydrocarbon group having 1 to 4 carbon atoms,

$R^2$ represents a linear or branched hydrocarbon group having 7 to 29 carbon atoms, containing no fluorine atoms, and optionally having an ether bond, and

X and Y each independently represent a direct bond or a divalent linking group.

[2] The compound according to [1] above, wherein in the general formula (1), X and Y represent direct bonds, and Rf is a linear or branched fluoroalkylene group containing 1 to 2 of at least any of a $CF_2$ unit, a CFH unit and a CFRf' unit.

[3] The compound according to [1] above wherein in the general formula (1), X and Y each independently represent a divalent linking group selected from the group consisting of $-SO_2-$, $-SO_2NR^3-$, $-NR^3SO_2-$, -O-, -C(=O)-O-, - O-C(=O)-, $-C(=O)-N(-R^3)-$, $-N(-R^3)-C(=O)-$, $-N(-R^3)-C(=O)-N(-R^3)-$, $-O-C(=O)-N(-R^3)-$, $-N(-R^3)-C(=O)O-$, and $-C_6H_4-$, wherein $R^3$ represents a hydrogen atom or a hydrocarbon group having 1 to 4 carbon atoms.

[4] The compound according to any one of [1] to [3] above, wherein in the general formula (1), L is represented by a general formula (L-1):

$$CH_2=CA-Z-* \quad ... \qquad (L-1)$$

wherein A represents a hydrogen atom, a hydrocarbon group having 1 to 4 carbon atoms, a fluorine atom, a chlorine atom, a bromine atom, or an iodine atom,

Z represents a divalent linking group, and

symbol * represents a bonding hand of L; and

n represents 1.

[5] The compound according to [4] above, wherein in the general formula (L-1), Z represents a divalent linking group selected from the group consisting of -C(=O)O-* and $-C(=O)NR^3-*$ wherein $R^3$ represents a hydrogen atom, or a hydrocarbon group having 1 to 4 carbon atoms.

[6] The compound according to any one of [1] to [3] above, wherein in the general formula (1), L is a carbon-containing linker moiety prepared by a process including reacting

(a) at least one isocyanate group-containing compound selected from the group consisting of isocyanate, diisocyanate and polyisocyanate, and

(b) at least one isocyanate reactive compound selected from the group consisting of compounds represented by general formulae (2a), (2b) and (2c):

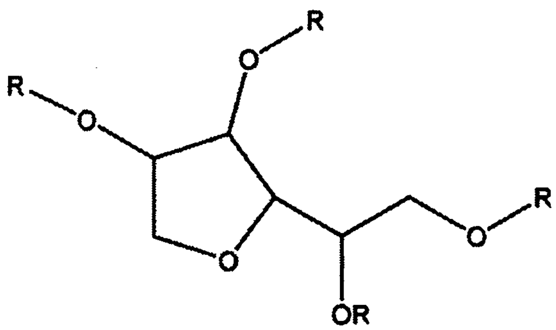

... (2a)

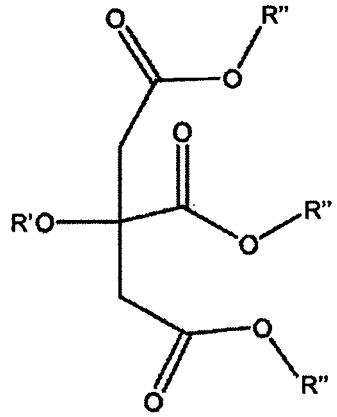

··· (2b)

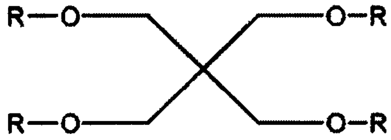

··· (2c)

wherein, in the general formulae (2a) and (2c),
each R independently represents -H, -*, -C(O)-*, - $(CH_2CH_2O)_p(CH(CH_3)CH_2O)_qH$, $-(CH_2CH_2O)_p(CH(CH_3)CH_2O)_q$-*, or - $(CH_2CH_2O)_p(CH(CH_3)CH_2O)_qC(O)$-*,
each p independently represents 0 to 20,
each q independently represents 0 to 20,
p+q is larger than 0,
symbol * represents a bonding hand of L, and
at least one R represents -H or - $(CH_2CH_2O)_p(CH(CH_3)CH_2O)_qH$, and at least another one R represents -*, -C(O)-*, $-(CH_2CH_2O)_p(CH(CH_3)CH_2O)_q$-*, or - $(CH_2CH_2O)_p(CH(CH_3)CH_2O)_qC(O)$-*, with the proviso that the isocyanate reactive compound is represented by the general formula (2a) or (2c) ; and
wherein, in the general formula (2b),
each R' independently represents -H, -*, -C(O)-*, - $(CH_2CH_2O)_{p'}(CH(CH_3)CH_2O)_{q'}H$, $-(CH_2CH_2O)_{p'}(CH(CH_3)CH_2O)_{q'}$-*, or $-(CH_2CH_2O)_{p'}(CH(CH_3)CH_2O)_{q'}C(O)$-*,
each R" independently represents -H, -*, - $(CH_2CH_2O)_{p'}(CH(CH_3)CH_2O)_{q'}H$, $-(CH_2CH_2O)_{p'}(CH(CH_3)CH_2O)_{q'}$-*, or $-(CH_2CH_2O)_{p'}(CH(CH_3)CH_2O)_{q'}C(O)$-*,
each p' independently represents 0 to 20,
each q' independently represents 0 to 20,
p'+q' is larger than 0,
symbol * is a bonding hand of L, and
at least one selected from R' and R" represents -H or $-(CH_2CH_2O)_{p'}(CH(CH_3)CH_2O)_{q'}H$, and at least another one selected from R' and R" represents -*, -C(O)-*, - $(CH_2CH_2O)_{p'}(CH(CH_3)CH_2O)_{q'}C(O)$or - $(CH_2CH_2O)_{p'}(CH(CH_3)CH_2O)_{q'}C(O)$-*, with the proviso that the isocyanate reactive compound is represented by the general formula (2b).

[7] The compound according to [6] above, wherein the diisocyanate and the polyisocyanate are at least one selected from the group consisting of hexamethylene diisocyanate homopolymer, 3-isocyanatemethyl-3,4,4-trimethylcyclohexyl isocyanate, bis-(4-isocyanatocyclohexyl)methane, and compounds represented by general formulae (3a), (3b), (3c) and (3d).

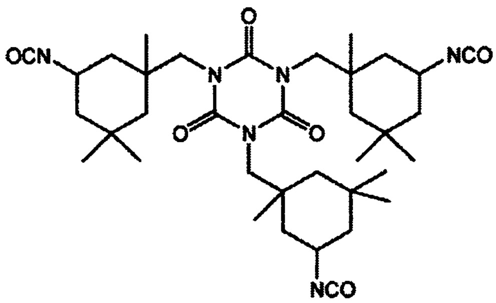

··· (3a)

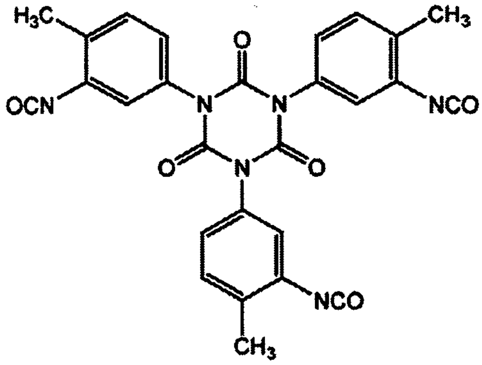

··· (3b)

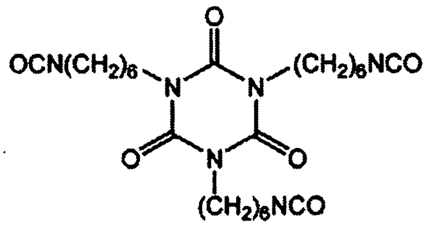

··· (3c)

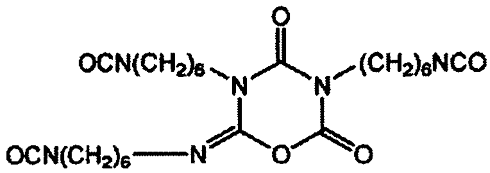

··· (3d)

[8] A polymer, comprising a repeating unit derived from the compound according to [4] or [5] above.
[9] An article having a surface formed of at least one selected from the group consisting of the compound according to any one of [1] to [7] above and the polymer according to [8] above.
[10] A composition, comprising:

at least one selected from the group consisting of the compound according to any one of [1] to [7] above and the polymer according to [8] above; and
a liquid medium.

[11] The composition according to [10] above, wherein the liquid medium is water or a mixture of water and an organic solvent.
[12] The composition according to [10] or [11] above, which is a water repellent.
[13] A textile product, which is processed with the composition according to any one of [10] to [12] above.

Advantageous Effects of Invention

According to the disclosure, a fluorine-containing compound capable of exhibiting high water-repellency despite the small number of fluorine atoms is provided. Further, according to the disclosure, a polymer, an article, a composition

and a textile product containing such a fluorine-containing compound are provided.

Brief Description of Drawings

[Fig. 1] Fig. 1 is a flow chart depicting each step of calculating solvation energy ΔG.
[Fig. 2] Fig. 2 is a graph of the correlation between contact angle of water (°) and solvation free energy ΔG (kcal/mol) with respect to water in polymers of a fluorine-containing compound.

Description of Embodiments

(Fluorine-containing compound)

The fluorine-containing compound of the disclosure is represented by the following general formula (1):

$$L\text{-}(R^1\text{-}X\text{-}Rf\text{-}Y\text{-}R^2)_n \ldots \qquad (1)$$

wherein L represents an n-valent organic group,
n represents an integer of 1 or more,
Rf represents a linear or branched fluoroalkylene group containing 1 to 4 of at least any of a $CF_2$ unit, a CFH unit and a CFRf' unit, and optionally having an ether bond, wherein Rf' represents a fluoroalkyl group having 1 carbon atom,
$R^1$ represents a direct bond, or a linear or branched hydrocarbon group having 1 to 4 carbon atoms,
$R^2$ represents a linear or branched hydrocarbon group having 7 to 29 carbon atoms, containing no fluorine atoms, and optionally having an ether bond, and
X and Y each independently represent a direct bond or a divalent linking group.

According to the fluorine-containing compound of the disclosure, high water-repellency (higher than that of a compound having -Rf at its end(s)) can be obtained despite the small number of fluorine atoms.

Rf represents a linear or branched fluoroalkylene group containing 1 to 4 of at least any of a $CF_2$ unit, a CFH unit and a CFRf' unit, and optionally having an ether bond, wherein Rf' represents a fluoroalkyl group having 1 carbon atom. These 3 types of units ($CF_2$ unit, CFH unit and CFRf' unit) are units focusing on a fluoromethylene group constituting the main chain of Rf (may be substituted or not substituted with Rf'). The number of at least any of the $CF_2$ unit, the CFH unit and the CFRf' unit is limited to 1 to 4, resulting in a small number of fluorine atoms (when the fluoroalkylene group of Rf contains 2 to 4 of at least any of these 3 types of units, 1 type or 2 or more types of these 3 types of units may be mixed in the fluoroalkylene group). Moreover, Rf does not represent a fluoroalkyl group present at an end of a compound, but represents a fluoroalkylene group present at a position closer to the acrylic structure, so that even higher water-repellency can be obtained. This is a unique finding obtained by the present inventors and differs from a conventional finding that a fluoroalkyl group present at an end of a compound can contribute to obtaining higher water-repellency. Rf' represents a fluoroalkyl group having one carbon atom, and can represent more specifically $-CF_3$, $-CF_2H$, $-CFH_2$ or the like. Rf can represent the one having no ether bond (-O-), however, high water-repellency can be obtained without substantially affecting water-repellency, even when Rf has an ether bond (-O-). More specifically, Rf can represent $-(CF_2)_n(CFH)_m(CFRf')_1-$ (n, m and 1 represent integers of 0 to 4, and satisfy $1 \leq n+m+1 \leq 4$, and the order of occurrence of the $CF_2$ unit, the CFH unit and the CFRf' unit is optional), or in the formula, an ether bond (-O-) may be present between any two units (adjacent to each other) selected from the $CF_2$ unit, the CFH unit and the CFRf' unit. Rf represents preferably $-(CF_2)_{n'}-$ (n' represents an integer of 1 to 4), $-(CF_2)_a-O-(CF_2)_{a'}-$ (a and a' represent integers of 0 to 4, and satisfy $1 \leq a+a' \leq 4$). Further, Rf may also contain at least any of the $CF_2$ unit, the CFH unit and the CFRf' unit, as well as contain, in addition to an ether bond, the $CH_2$ unit, any suitable substituent, heteroatoms or the like (e.g., -S-) other than oxygen in some cases, if they are present.

$R^1$ represents a direct bond, or a linear or branched hydrocarbon group having 1 to 4 carbon atoms, preferably a linear or branched hydrocarbon group having 1 to 2 carbon atoms, further preferably a methylene group or an ethylene group, and particularly preferably a methylene group.

$R^2$ represents a linear or branched hydrocarbon group having 7 to 29 carbon atoms, containing no fluorine atoms, and optionally having an ether bond. Regarding $R^2$, a linear or branched hydrocarbon group having 7 to 29 carbon atoms, containing no fluorine atoms can be a linear or branched alkyl group having 7 to 29 carbon atoms, containing no fluorine atoms. $R^2$ can represent the one having no ether bond (-O-), however, high water-repellency can

be obtained without substantially affecting water-repellency, even when $R^2$ has an ether bond (-O-). More specifically, $R^2$ can represent $-(CH_2)_c-O_d-(CH_2)_eCH_3$ (d represents 0 or 1, and c and e represent integers satisfying c+e = an integer of 7 to 29), for example.

X and Y each independently represent a direct bond or a divalent linking group. Divalent linking group(s) corresponding to X and/or Y is not limited, and can be selected from the group consisting of $-SO_2-$, $-SO_2NR^3-$, - $NR^3SO_2-$, -O-, -C(=O)-O-, -O-C(=O)-, -C(=O)-N(-$R^3$)-, -N(-$R^3$)-C(=O)-, -N(-$R^3$)-C(=O)-N(-$R^3$)-, -O-C(=O)-N(-$R^3$)-, -N(-$R^3$)-C(=O)O-, and $-C_6H_4-$ (or disubstituted benzene), for example. If present, $R^3$ represents a hydrogen atom, or a hydrocarbon group having 1 to 4 carbon atoms. Regarding $R^3$, a hydrocarbon group having 1 to 4 carbon atoms can be a linear or branched alkyl group having 1 to 4 carbon atoms, and preferably a methyl group, for example. $R^3$ represents preferably a hydrogen atom. X and Y represent preferably direct bonds or -O-, and particularly preferably direct bonds.

In a preferable embodiment, X and Y can represent direct bonds, and Rf can represent a linear or branched fluoroalkylene group (having no ether bond) containing 1 to 2 of at least any of the $CF_2$ unit, the CFH unit and the CFRf' unit.

L represents an n-valent organic group, and n represents an integer of 1 or more. In other words, in the fluorine-containing compound of the disclosure, -$R^1$-X-Rf-Y-$R^2$ is bound to each of n bonding hands (in the present disclosure, may be represented by symbol *, and n denotes the number of bonding hands) possessed by L ($R^1$ is bound to the bonding hand of L). The value (number) of n can differ according to L. For example, when the fluorine-containing compound is a polymer (described later), the value of n can be increased correspondingly, and no particular upper limit is present.

For example, L is represented by the following general formula (L-1):

$CH_2$=CA-Z-* ... (L-1)

wherein A represents a hydrogen atom, a hydrocarbon group having 1 to 4 carbon atoms, a fluorine atom, a chlorine atom, a bromine atom, or an iodine atom,
Z represents a divalent linking group, and
symbol * represents a bonding hand of L; and
n may represent 1.

In this case, the fluorine-containing compound is represented by the following general formula (1a).

$CH_2$=CA-Z-$R^1$-X-Rf-Y-$R^2$ ... (1a)

The fluorine-containing compound represented by general formula (1a) can be understood as a polymerizable monomer. According to such a fluorine-containing compound, a polymer exhibiting sufficiently high water-repellency can be obtained despite the small number of fluorine atoms.

More specifically, the fluorine-containing compound represented by general formula (1a) can be understood as an acrylic monomer optionally substituted at position $\alpha$. In general formula (1a), A represents a hydrogen atom, a hydrocarbon group having 1 to 4 carbon atoms, a fluorine atom, a chlorine atom, a bromine atom, or an iodine atom. Regarding A, the hydrocarbon group having 1 to 4 carbon atoms can be a linear or branched alkyl group having 1 to 4 carbon atoms, preferably a methyl group, for example. A represents preferably a hydrogen atom or a methyl group.

Examples of a divalent linking group corresponding to Z are not limited and can be selected from the group consisting of -C(=O)O-* and -C(=O)N$R^3$-* ($R^3$ represents a hydrogen atom or a hydrocarbon group having 1 to 4 carbon atoms).

The fluorine-containing compound represented by general formula (1a) can be synthesized by any appropriate method. For example, the fluorine-containing compound represented by general formula (1) may be synthesized from raw-material compounds represented by the following general formulae (p) and (q), and specifically, by binding reaction between X' and X":

$CH_2$=CA-Z-$R^1$-X' ... (p)

X"-Rf-Y-$R^2$ ... (q)

wherein X' and X" are a monovalent organic group being a reaction precursor of X, which can be selected according to X.

Furthermore, for example, L may be a carbon-containing linker moiety prepared by a process including reacting:

(a) at least one isocyanate group-containing compound selected from the group consisting of isocyanate, diisocyanate and polyisocyanate (hereinafter, may also be simply referred to as "isocyanate group-containing compound"),

and

(b) at least one isocyanate reactive compound selected from the group consisting of compounds represented by the following general formulae (2a), (2b) and (2c) (hereinafter, may also be simply referred to as "isocyanate reactive compound");

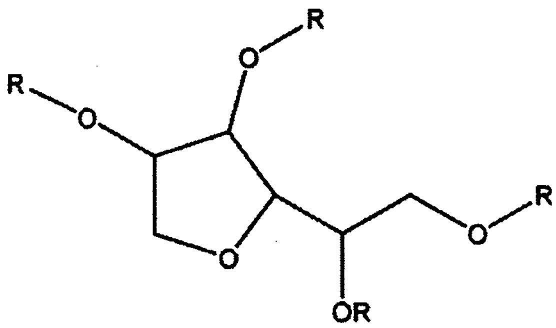

··· (2a)

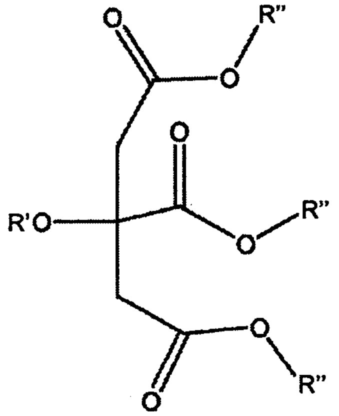

··· (2b)

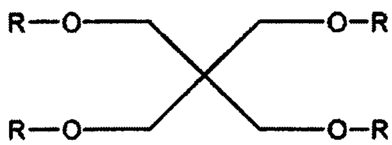

··· (2c)

wherein, in general formulae (2a) and (2c),
each R independently represents -H, -*, -C(O)-*, - $(CH_2CH_2O)_p(CH(CH_3)CH_2O)_qH$, $-(CH_2CH_2O)_p(CH(CH_3)CH_2O)_q$-*, or - $(CH_2CH_2O)_p(CH(CH_3)CH_2O)_qC(O)$-*,
each p independently represents 0 to 20,
each q independently represents 0 to 20,
p+q is larger than 0,
symbol * represents a bonding hand of L,
at least one R represents -H or - $(CH_2CH_2O)_p(CH(CH_3)CH_2O)_qH$, and at least another one R represents -*, -C(O)-*, $-(CH_2CH_2O)_p(CH(CH_3)CH_2O)_q$-*, or - $(CH_2CH_2O)_p(CH(CH_3)CH_2O)_qC(O)$-*, with the proviso that the isocyanate reactive compound is represented by general formula (2a) or (2c), and
wherein, in general formula (2b),
each R' independently represents -H, -*, -C(O)-*, - $(CH_2CH_2O)_{p'}(CH(CH_3)CH_2O)_{q'}H$, $-(CH_2CH_2O)_{p'}(CH(CH_3)CH_2O)_{q'}$-*, or $-(CH_2CH_2O)_{p'}(CH(CH_3)CH_2O)_{q'}C(O)$-*,
each R" independently represents -H, -*, - $(CH_2CH_2O)_{p'}(CH(CH_3)CH_2O)_{q'}H$, $-(CH_2CH_2O)_{p'}(CH(CH_3)CH_2O)_{q'}$-*, or $-(CH_2CH_2O)_{p'}(CH(CH_3)CH_2O)_{q'}C(O)$-*,
each p' independently represents 0 to 20,
each q' independently represents 0 to 20,
p'+q' is larger than 0,
symbol * is a bonding hand of L, and

at least one selected from R' and R" represents -H or $(CH_2CH_2O)_{p'}(CH(CH_3)CH_2O)_{q'}H$, and at least another one selected from R' and R" represents -*, -C(O)-*, - $(CH_2CH_2O)_{p'}(CH(CH_3)CH_2O)_{q'}$-* or -$((CH_2CH_2O)_{p'}(CH(CH_3)CH_2O)_{q'}C(O)$-*, with the proviso that the isocyanate reactive compound is represented by general formula (2b).

Note that p, q, p' and q' each represent an integer of 0 to 20 in only one fluorine-containing compound, but can be represented by a mean value in the case of an assembly of a plurality of fluorine-containing compounds.

In this case, L in the fluorine-containing compound(s) represents a carbon-containing linker moiety prepared by a process including reacting isocyanate group-containing compound (a) with isocyanate reactive compound (b). Such a carbon-containing linker moiety can be usually polyvalent, but is not limited thereto. Isocyanate reactive compound (b) has at least one -OH group when represented by general formula (2a) or (2c), and has at least one -OH group or -COOH group when represented by general formula (2b). Hence, through reaction of isocyanate group-containing compound (a) with isocyanate reactive compound (b), a reaction product can be obtained, in which these compounds are bound via a urethane bond or an amide bond. Such reaction is a known reaction and can be carried out under any appropriate conditions.

In such fluorine-containing compound, $-R^1$-X-Rf-Y-$R^2$ is bound to each of n bonding hands (represented by symbol *, and n denotes the number of bonding hands) present at a portion derived from isocyanate reactive compound (b) ($R^1$ is bound to the bonding hand of L). The $-R^1$-X-Rf-Y-$R^2$ portion is bound to a bonding hand (represented by symbol *) present in isocyanate reactive compound (b) before reaction. Isocyanate reactive compound (b) can be one type or a mixture of any two or more types selected from the group consisting of compounds represented by general formulae (2a), (2b) and (2c), and of these, is preferably the compound represented by general formula (2a).

Isocyanate group-containing compound (a) can be one type or a mixture of any two or more types selected from the group consisting of isocyanate, diisocyanate and polyisocyanate. When isocyanate group-containing compound (a) is diisocyanate and/or polyisocyanate, and isocyanate reactive compound (b) has a total of two or more -OH groups and/or COOH groups, a reaction product obtained from these compounds may be a polymer in some cases, but is not limited thereto.

Diisocyanate and polyisocyanate can be at least one type selected from the group consisting of preferably, hexamethylene diisocyanate homopolymer, 3-isocyanatemethyl-3,4,4-trimethylcyclohexyl isocyanate, bis-(4-isocyanatocyclohexyl)methane, and compounds represented by the following general formulae (3a), (3b), (3c) and (3d).

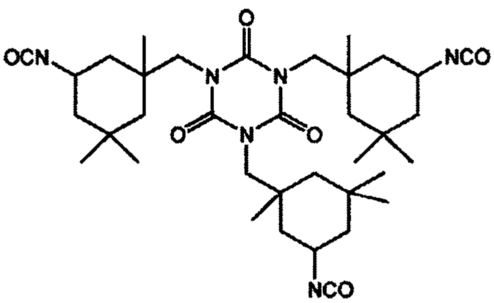

··· (3a)

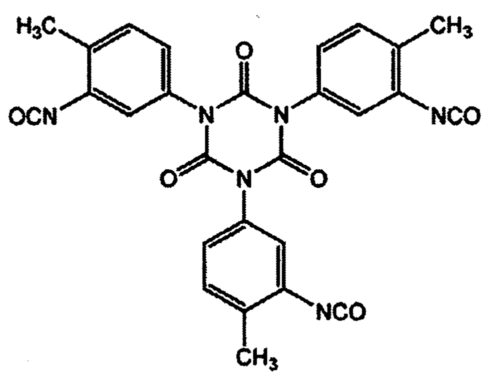

··· (3b)

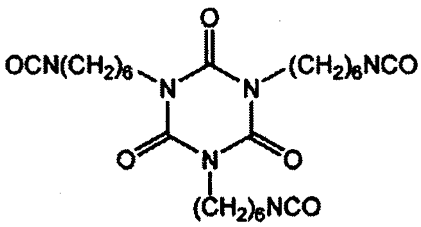

··· (3c)

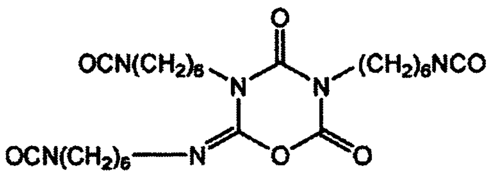

··· (3d)

The compounds represented by general formulae (3a), (3b), (3c) and (3d) are understood as diisocyanate trimers. A diisocyanate trimer can be one type or a mixture of any two or more types selected from the group consisting of the compounds represented by general formulae (3a), (3b), (3c) and (3d), and of these, is preferably the compound represented by general formula (3c) .

(Polymer)

The polymer of the disclosure contains repeating units derived from the above fluorine-containing compound represented by general formula (1a) (polymerizable monomer).

Note that such polymer of the disclosure can also be understood as a form of the fluorine-containing compound represented by general formula (1) of the disclosure. For example, L is represented by the following general formula (L-1'):

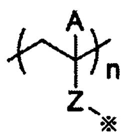

· · · (L−1')

wherein A represents a hydrogen atom, a hydrocarbon group having 1 to 4 carbon atoms, a fluorine atom, a chlorine atom, a bromine atom, or an iodine atom, Z represents a divalent linking group, and symbol * represents a bonding hand of L,

wherein n represents the number of repeating units derived from the fluorine-containing compound represented by general formula (1a) (polymerizable monomer), and may be the number that agrees with n in general formula (1).

The polymer of the disclosure exhibits sufficiently high water-repellency despite the use of the fluorine-containing compound represented by general formula (1a) having a small number of fluorine atoms.

The polymer of the disclosure may further contain repeating units derived from another polymerizable monomer. Such another polymerizable monomer preferably has no fluoroalkyl group and no fluoroalkylene group.

For example, another polymerizable monomer can be a compound represented by the following general formula (b) :

$CH_2$=CB-T ... (b)

wherein B represents a hydrogen atom, a methyl group, a fluorine atom, a chlorine atom, a bromine atom, or an iodine atom,

T represents a hydrogen atom, a chlorine atom, a bromine atom, an iodine atom, a chain or cyclic hydrocarbon group having 1 to 40 carbon atoms, or a chain or cyclic organic group having an ester bond and having 2 to 41 carbon atoms.

The compound (polymerizable monomer) represented by general formula (b) can be understood as an acrylic monomer optionally substituted at position α, and can be copolymerized with the compound (polymerizable monomer) represented by general formula (1a). In general formula (b), B represents a hydrogen atom, a methyl group, a fluorine

atom, a chlorine atom, a bromine atom, or an iodine atom, and preferably a hydrogen atom or a methyl group. T represents a hydrogen atom, a chlorine atom, a bromine atom, an iodine atom, a chain or cyclic hydrocarbon group having 1 to 40 carbon atoms, or a chain or cyclic organic group having an ester bond and having 2 to 41 carbon atoms.

The polymer of the disclosure can contain repeating units derived from the compound represented by general formula (1a) in an amount ranging from 2 to 100% by mass, preferably 10 to 100% by mass, and more preferably 30 to 100% by mass based on the entire polymer. In the case of 100% by mass, the polymer is a homopolymer of the compounds (polymerizable monomers) represented by general formula (1a). In the case of less than 100% by mass, the polymer is a copolymer of the compound (polymerizable monomer) represented by general formula (1a) and another polymerizable monomer (typically the compound represented by general formula (b)). The copolymer is not limited and an example thereof can be a random, block or graft copolymer.

In the case of homopolymer, its melting point originates from the crystallinity of an Rf group, and can be measured by Differential Scanning Calorimetry (DSC). The presence of the Rf group crystal is also confirmed by observing a peak derived from its self-aggregation by wide-angle X-ray diffraction. Usually, the crystallinity is represented by the degree of crystallinity that is found by the following formula.

$$[\text{Degree of crystallinity (\%)}] = I_{Rf}/(I_{Rf}+I_{am}) \times 100$$

Here, $I_{Rf}$ represents the peak intensity of the Rf group appearing at 2θ=18°, and $I_{am}$ represents the peak intensity of the amorphous region.

The weight-average molecular weight of the polymer of the disclosure may range from 1,000 to 500,000, and preferably 5,000 to 200,000. If the weight-average molecular weight is 1,000 or more, high durability is obtained in the form of a film, and if the weight-average molecular weight is 500,000 or less, manageable viscosity is obtained in the form of a composition. The weight-average molecular weight is the value found by gel permeation chromatography in terms of polystyrene. The weight-average molecular weight can also be regulated using a chain transfer agent.

The polymer of the disclosure can be produced by an appropriate method employed depending on raw materials (compound/polymerizable monomer) to be used, with the use of the compound represented by general formula (1a) and another polymerizable monomer if necessary. As a polymerization method, for example, massive polymerization, solution polymerization, emulsion polymerization, radiation-induced polymerization or the like can be applied. An example of a method that can be employed is a method that involves dissolving raw materials (compound/polymerizable monomer) in an appropriate organic solvent, causing solution polymerization to take place by the action of a polymerization initiation source (a peroxide soluble in an organic solvent to be used, an azo compound or ionizing radiation, etc.). Specifically, peroxides, various types of azo-based or persulfuric acid-based compound can be used as polymerization initiators for reaction systems. Furthermore, a method that can be employed involves emulsifying a mixture of compounds to be copolymerized in water in the presence of a surfactant, followed by stirring for copolymerization.

The polymer of the disclosure can be prepared according to an ordinary method in any form such as a liquid composition (obtained by dissolving, dispersing or suspending the polymer in a liquid medium, etc.) and an aerosol, and thus can be prepared in the form of a water repellent.

(Article)

The article of the disclosure has a surface formed of at least one selected from the group consisting of the above fluorine-containing compound of the disclosure and the above polymer of the disclosure (hereinafter, referred to as "the fluorine-containing compound of the disclosure or the like").

The article of the disclosure can have a water-repellent surface.

Such surface can be applied to an object to be processed by any appropriate method in accordance with the type of the object to be processed, the prepared form (composition, aerosol etc.) of the fluorine-containing compound, or the like. For example, in the case of a composition, a method that can be employed involves adhering the composition to a surface of an object to be processed by a known coating process such as immersion coating, and drying the surface. Moreover, the composition may be applied together with an appropriate cross-linking agent as necessary, so as to perform curing. For example, in the case of aerosol, the aerosol may be simply sprayed to blow it on an object to be processed, and then the product is dried immediately after spraying, so that sufficient water-repellency can be exhibited.

The object to be processed (and by extension the resulting article after processing) is not limited, and can be made of any appropriate material. Examples thereof can include fiber, stone, paper, wood, leather, metal, ceramics, glass, and plastic described below.

(Composition)

The composition of the disclosure contains the above fluorine-containing compound of the disclosure or the like (at least one selected from the group consisting of the compound of the above disclosure and the polymer of the above disclosure) and a liquid medium.
The composition of the disclosure is applied to an object to be processed, so that the composition can impart water-repellency to the object to be processed. More specifically, the composition of the disclosure can be understood as a form of a water repellent, and the fluorine-containing compound of the disclosure or the like is an active ingredient of a water repellent.
The composition of the disclosure can contain the fluorine-containing compound of the disclosure or the like, in an amount ranging from 1 to 99% by mass, preferably 5 to 80% by mass, and more preferably 5 to 50% by mass based on the entire composition.
In the composition of the disclosure, the fluorine-containing compound of the disclosure or the like may be dissolved, dispersed or suspended in a liquid medium. The composition of the disclosure may be a liquid composition that can be a solution, a dispersion or a suspension (e.g., emulsion).
The liquid medium can be water or a mixture of water and an organic solvent. In this case, the composition of the disclosure can be understood as an aqueous composition.
Examples of an organic solvent to be mixed with water include alcohols such as methanol, ethanol, 1-propanol, isopropanol, and butanol. In the mixture of water and an organic solvent, the mass ratio of water : organic solvent can be 1:99 to 99:1.
However, the liquid medium is not limited to water or a mixture of water and an organic solvent, and may be an organic solvent, for example. Examples of the organic solvent include ketones such as acetone, methyl ethyl ketone, and methyl isobutyl ketone, esters such as ethyl acetate, propyl acetate, and butyl acetate, alcohols such as ethanol, isopropanol, butanol, 1,3-butanediol, and 1,5-pentanediol, halogenated hydrocarbons such as chloroform, perchloroethylene, trichlene, 1,1-dichloro-2,2,3,3,3-pentafluoropropane, 1,3-dichloro-1,2,2,3,3-pentafluoropropane, and 1,1-dichloro-1-fluoroethane (HCFC-141b), hydrocarbons such as octane, petroleum, toluene, and xylene, dipropylene glycol, dipropylene glycol monomethyl ether, tripropylene glycol monomethyl ether, polypropylene glycol, triethylene glycol dimethyl ether, propylene glycol, and ethylene glycol.
The composition of the disclosure may also contain other components, such as a surfactant (e.g., anion surfactant, cation surfactant and/or nonionic surfactant), if appropriate.
Examples of anion surfactants include sodium lauryl sulfate, triethanolamine lauryl sulfate, sodium polyoxyethylene lauryl ether sulfate, sodium polyoxyethylene nonylphenyl ether sulfate, triethanolamine polyoxyethylene lauryl ether sulfate, sodium cocoyl sarcosin, sodium N-cocoyl methyl taurine, sodium polyoxyethylene coconut alkyl ether sulfate, sodium diether hexyl sulfosuccinate, sodium $\alpha$-olefin sulfonate, sodium lauryl phosphate, sodium polyoxyethylene lauryl ether phosphate, and perfluoroalkyl carboxylate.
Examples of cation surfactants include dialkyl (C12-C22) dimethylammonium chloride, alkyl (coconut) dimethylbenzylammonium chloride, octadecylamine acetate, tetradecylamine acetate, beef fat alkylpropylene diamine acetate, octadecyltrimethylammonium chloride, alkyl (beef fat) trimethylammonium chloride, dodecyltrimethylammonium chloride, alkyl (coconut) trimethylammonium chloride, hexadecyltrimethylammonium chloride, biphenyltrimethylammonium chloride, alkyl (beef fat) imidazoline quaternary salt, tetradecylmethylbenzylammonium chloride, octadecyldimethylbenzylammonium chloride, dioleyldimethylammonium chloride, polyoxyethylene dodecylmonomethylammonium chloride, polyoxyethylenealkyl (C12-C22) benzylammonium chloride, polyoxyethylene laurylmonomethylammonium chloride, 1-hydroxyethyl-2-alkyl (beef fat) imidazoline quaternary salt, a silicone-based cation surfactant having a siloxane group as a hydrophobic group, and a fluorine-based cation surfactant having a fluoroalkyl group as a hydrophobic group.
Examples of nonionic surfactants include polyoxyethylene lauryl ether, polyoxyethylene tridecyl ether, polyoxyethylene cetyl ether, polyoxyethylene polyoxypropylene cetyl ether, polyoxyethylene stearyl ether, polyoxyethylene oleyl ether, polyoxyethylene nonylphenyl ether, polyoxyethylene octylphenyl ether, polyoxyethylene monolaurate, polyoxyethylene monostearate, polyoxyethylene monooleate, sorbitan monolaurate, sorbitan monostearate, sorbitan monopalmitate, sorbitan monostearate, sorbitan monooleate, sorbitan sesquioleate, sorbitan trioleate, polyoxyethylene sorbitan monolaurate, polyoxyethylene sorbitan monopalmitate, polyoxyethylene sorbitan monostearate, polyoxysorbitan monooleate, polyoxyethylene polyoxypropylene block polymer, polyglycerin fatty acid ester, polyether-modified silicone oil (trade names: SH3746, SH3748, SH3749, SH3771 (manufactured by Dow Corning Toray Silicone Co. Ltd.)), perfluoroalkyl ethylene oxide adducts, fluoroalkyl ethylene oxide adducts, and perfluoroalkyl oligomers.

(Textile product)

The textile product of the disclosure is processed with the above composition of the disclosure.
The processed surface of the textile product of the disclosure can exhibit water-repellency.

Various examples can be given as textile products. Examples thereof include animal and vegetable natural fibers such as cotton, hemp, wool and silk, synthetic fibers such as polyamide, polyester, polyvinyl alcohol, polyacrylonitrile, polyvinyl chloride and polypropylene, semi-synthetic fibers such as rayon and acetate, inorganic fibers such as glass fibers, carbon fibers, and asbestos fibers, or mixed fibers thereof. The textile product may be in any form such as fiber, thread, and fabric.
The composition of the disclosure can be diluted as appropriate and used upon processing in such a manner that the content of the fluorine-containing compound of the disclosure ranges from 0.1 to 30% by mass, and preferably 1 to 10% by mass. Such appropriate dilution of the fluorine-containing compound in such a manner that the content is within the above range enables to prevent the deterioration of the texture of the textile product while appropriately imparting water-repellency to the textile product.

Examples

(Synthesis example of compound)

Compounds A1, A2, and Z depicted in Table 1 were synthesized.

[Table 1]

| Compound | Chemical formula |
|---|---|
| A1* | $CH_2$=CH-C(=O)-O-$CH_2$-$CF_2$-$CH_2$-O-$CH_2)_5CH_3$ |
| A2 | $CH_2$=CH-C(=O)-O-$CH_2$-O-$(CH_2)_6$-$CF_3$ |
| Z | $CH_2$=CH-C(=O)-O-$CH_2CH_2$-$(CF_2)_5CF_3$ |

Compound A1 is an example of the fluorine-containing compound of the disclosure, and compound A2 and compound Z are comparative examples (in Table 1, the example of the fluorine-containing compound of the disclosure is indicated with symbol*, and the same applies to those in the following tables.). In compound A1, the number of $CF_2$ units of Rf is 1, and the number of carbon atoms of $R^2$ is 7 (having an ether bond). Compound A2 has an inverted structure corresponding to that of compound A1. Specifically, in compound A2, the number of $CF_2$ ($CF_3$ at an end of a fluoroalkyl group) units of a fluoroalkyl group at an end of the compound is 1, the number of carbon atoms of a hydrocarbon group located closer to the acrylic structure and containing no fluorine atoms is 7 (having an ether bond). Compound Z is a conventionally representative compound exhibiting water-repellency. In compound Z, the number of $CF_2$ ($CF_3$ at an end of the fluoroalkyl group) units of a fluoroalkyl group at an end of the compound is 6, and the number of carbon atoms of a hydrocarbon group located closer to the acrylic structure and containing no fluorine atoms is 2 (no ether bond).
A procedure for the synthesis of compounds A1 and A2 is as follows. Note that compound Z is known and thus the explanation of the procedure for synthesis is omitted.

- Compound A1

A reaction vessel was charged with 1-hexanol (10 g) and 1,2-dimethoxyethane and then cooled. 60% NaH (4.3 g) was added to the thus obtained mixture (mixed solution), and then the mixture was stirred at room temperature. 2,2,3,3-Tetrafluorooxetane (6.4 g) was added while cooling the mixture and then the temperature was increased to room temperature, followed by stirring overnight. Water was added to the thus obtained reaction solution to extract a reaction product, and then the reaction product was washed and then dehydrated. The organic solvent was distilled off under reduced pressure to obtain 18 g of a crude product. Purification was carried out to obtain 10 g of compound (i).
A reaction vessel was charged with diethyl ether and compound (i) (10 g) and then cooled. A 1.0 M LAH (lithium aluminum hydroxide) diethyl ether solution (98 mL) was added dropwise to the thus obtained mixture (mixed solution), and then the temperature was increased to room temperature. The thus obtained reaction solution was cooled, and then sodium sulfate decahydrate was added. The filtrate (diethyl ether solution) was concentrated under reduced pressure to obtain about 9.0 g of a crude product. Purification was carried out to obtain 6.0 g of compound (ii) having a gas chromatography analysis purity of 96% by mass.
A reaction vessel was charged with THF (tetrahydrofuran), compound (ii) (6.0 g) and triethylamine (6.0 g), and then cooled with stirring. Acryloyl chloride (3.1 g) was added dropwise to the mixture (mixture solution), and then the temperature was increased to room temperature. Water was added to the thus obtained reaction solution to extract a reaction product, and then the reaction product was washed and then dehydrated. The organic solvent was distilled off under reduced pressure to obtain about 6.0 g of a crude product. Purification was carried out to obtain 3.0 g of compound

A1.

- Compound A2

A reaction vessel was charged with 1,2-dimethoxyethane and 2,2,2-trifluoroethanol (5.5 g) and then cooled. 60% NaH (2.4 g) was added to the thus obtained mixture (mixed solution), and then the mixture was stirred at room temperature. 6-Chloro-1-hexanol (5.0 g) was added to this mixture, and then the mixture was heated and stirred at 83°C (reflux). Then a sodium 2,2,2-trifluoroethoxide solution was added. Water (50 mL) was added to the reaction solution to extract a reaction product, and then the reaction product was washed and then dehydrated. The organic solvent was distilled off under reduced pressure to obtain about 6.3 g of a crude product. Purification was carried out to obtain 4.0 g of 6-(2,2,2-trifluoroethoxy) hexanol.

A reaction vessel was charged with THF, 6-(2,2,2-trifluoroethoxy) hexanol (4.0 g) and triethylamine (4.0 g), and then cooled with stirring. Acryloyl chloride (2.0 g) was added to the mixture (mixture solution), and then the temperature was increased to room temperature. Water was added to the thus obtained reaction solution to extract a reaction product, and then the reaction product was washed and then dehydrated. The organic solvent was distilled off under reduced pressure to obtain about 4.4 g of a crude product. Purification was carried out to obtain 1.6 g of compound A2.

(Production example of polymer)

A polymer (homopolymer) was produced by polymerizing compound A1. The production procedure was as follows.

A reaction vessel was charged with 1.0 g of compound A1, 4.0 g of toluene was added to dissolve compound A1, the inside of the reaction flask was replaced with nitrogen, and then 0.007 g of 2,2-azobisisobutyronitrile was added. A reaction was carried out at 65°C overnight to obtain a crude polymer. The crude polymer was reprecipitated to obtain a purified polymer (polymer of compound A1) as a solid.

The weight-average molecular weight of the polymer of compound A1 was 32,000 (in terms of polystyrene).

The polymer (homopolymer) of compound A2 and that of compound Z were produced in the same manner as described above. The weight-average molecular weights of the polymer of compound A2 and that of compound Z were 28,000 and 42,000 (in terms of polystyrene).

(Evaluation of water-repellency: Measurement of contact angle of water)

The contact angle of water of each of polymers (homopolymers) obtained from compounds A1, A2 and Z was measured. Specifically, a solution containing 1% by mass of the polymer in chloroform was prepared. The solution was placed on a silicon substrate, and then spin-coated at 2000 rpm for 25 seconds to prepare a film. Water was dropped on the obtained film and then the contact angle was measured. The contact angle was measured using a contact angle measurement apparatus (manufactured by Kyowa Interface Science Co., Ltd.) and 2 μL of water in an environment at 25°C. Table 2 depicts the measurement results of the contact angle of water (°).

[Table 2]

| Compound | Contact angle of water (°) | ΔG (kcal/mol) |
|---|---|---|
| A1* | 107.1 | -0.97 |
| A2 | 103.3 | -1.52 |
| Z | 105.5 | -1.49 |

With reference to Table 2, the polymer of compound A1 exhibited a larger contact angle and sufficiently high water-repellency compared with the polymer of compound Z. On the other hand, the polymer of compound A2 exhibited a smaller contact angle and lower water-repellency than those of the polymer of compound A1 and that of compound Z.

(Evaluation of water-repellency: ΔG calculation by simulation)

For a polymer (homopolymer) obtained from a compound, the solvation energy ΔG (kcal/mol) with respect to water can be calculated by simulation. Specifically, ΔG is calculated by the following steps S1 to S4 with reference to Fig. 1 using an appropriate calculation device (including an input device, an output device, a CPU, a memory, etc.).

- Step S1

With water (small molecule) defined as a solute and a polymer (homopolymer) defined as a solvent, data of the system is acquired in step S1. The system is an objective solution system consisting of a solute and a solvent. The data includes information on the types of multiple atoms constituting the solute and the solvent, and the force field of each atom, and further includes the initial position of each atom, the initial velocity of each atom, and the like. Regarding the solvent molecule (polymer), information on the classification of a polymer, for example, information on the homopolymerization system, crystallinity, molecular weight and the like is also included in these data. In addition, conditions for the solvent molecule (polymer) to absorb the solute molecule (water), such as temperature and pressure, are also included in these data.

- Step S2

Subsequently, molecular dynamics simulations are used to calculate the motion of each atom contained in each of the solute and the solvent. As a result, the coordinates of each atom at a plurality of times from the start to the end of the calculation are acquired.

- Step S3

Subsequently, based on the coordinate of each atom at each time, the energy distribution function of the solute and the solvent to be generated is calculated from the following formula (c).

$$\rho(\varepsilon) = \sum_{i} \delta(\varepsilon - \nu(\psi, xi)) \quad \cdots (c)$$

In the formula,

ε represents the interaction energy between the solute and the solvent,
ρ represents a distribution function,
φ represents the coordinate of the solute,
xi represents the coordinate of the i-th solvent,
ν (φ, xi) represents the interaction potential between the solute and the solvent, and
δ is a delta function.

The above formula (c) represents the distribution function ρ with the interaction energy ε of the solute and the solvent on the horizontal axis, and represents the instantaneous distribution of the solvent around the solute. Specifically, the above formula (c) represents a histogram of the interaction energy of the pair of the solute and the solvent in the instantaneous arrangement of the objective system. By focusing on the interaction energy without directly dealing with the details of the molecular structure, the solute molecule and the solvent molecule are regarded as one unit as a whole. The above equation (c) is calculated with each sampled instantaneous arrangement, and the energy distribution function (statistical average <ρ> of the above distribution function ρ) is found from the statistical average (the average of the above (c) at all moments). The energy distribution function is calculated only in the state "before" and the state "after" the introduction of the solute into the solvent. Therefore, calculating the energy distribution function is equivalent to performing a molecular dynamics simulation only in the state "before" and the state "after" the introduction of the "solute" into the "solvent".

- Step S4

Subsequently, the solvation free energy ΔG is calculated using the energy distribution function calculated in step S3 and the free energy functional. Specifically, the energy distribution function calculated in step S3 is substituted into the free energy functional prepared in advance. The free energy functional is used for approximately deriving the solvation free energy from the energy distribution function. As the free energy functional, the one formulated in Non Patent Literature 1 is used.

As described above, the solvation energy ΔG (kcal/mol) with respect to water of each of polymers (homopolymers) obtained from the compounds A1, A2 and Z was calculated. Table 2 also depicts the calculation results of ΔG.

Further, from the results in Table 2, a graph of the correlation between the contact angle (°) of water and the solvation free energy ΔG (kcal/mol) with respect to water is depicted in Fig. 2.

The polymer of the fluorine-containing compound is understood as exhibiting a high correlation between the contact angle of water and ΔG, as depicted in Fig. 2. Further, as understood from Table 1 and Fig. 2, ΔG should be -1 kcal/mol or more in order to obtain water-repellency as high as that of compound A (contact angle of water as large as that of compound A).

Further, regarding the polymers (homopolymers) obtained from compounds B1 to G1 and B2 to G2 depicted in Tables 3 and 4, the solvation energy ΔG (kcal/mol) with respect to water was calculated in the same manner as above. Table 5 depicts the calculation results of ΔG. In addition, Tables 3 to 5 present the results of compounds A1 and A2 again. Compounds A2 to G2 have inverted structures corresponding to those of compounds A1 to G1, and.

[Table 3]

| Compound | Chemical formula | Number of $CF_2$ units of Rf | Number of carbon atoms of $R^2$ (ether bond) |
|---|---|---|---|
| A1* | $CH_2$=CH-C(=O)-O-$CH_2$-$CF_2$-$CH_2$-O-$(CH_2)_5CH_3$ | 1 | 7 (with ether bond) |
| B1* | $CH_2$=CH-C(=O)-O-$CH_2$-$CF_2$-$(CH_2)_6CH_3$ | 1 | 7 (no ether bond) |
| C1* | $CH_2$=CH-C(=O)-O-$CH_2$-$CF_2$-$(CH_2)_{14}CH_3$ | 1 | 15 (no ether bond) |
| D1* | $CH_2$=CH-C(=O)-O-$CH_2$-$CF_2$-$(CH_2)_{28}CH_3$ | 1 | 29 (no ether bond) |
| E1 | $CH_2$=CH-C(=O)-O-$CH_2$-$CF_2$-$(CH_2)_2CH_3$ | 1 | 3 (no ether bond) |
| F1 * | $CH_2$=CH-C(=O)-O-$CH_2$-$(CF_2)_2$-$(CH_2)_6CH_3$ | 2 | 7 (no ether bond) |
| G1* | $CH_2$=CH-C(=O)-O-$CH_2$-$(CF_2)_4$-$(CH_2)_6CH_3$ | 4 | 7 (no ether bond) |

[Table 4]

| Compound | Chemical formula |
|---|---|
| A2 | $CH_2$=CH-C(=O)-O-$CH_2$-O-$(CH_2)_6$-$CF_3$ |
| B2 | $CH_2$=CH-C(=O)-O-$(CH_2)_7$-$CF_3$ |
| C2 | $CH_2$=CH-C(=O)-O-$(CH_2)_{15}$-$CF_3$ |
| D2 | $CH_2$=CH-C(=O)-O-$(CH_2)_{29}$-$CF_3$ |
| E2 | $CH_2$=CH-C(=O)-O-$(CH_2)_3$-$CF_3$ |
| F2 | $CH_2$=CH-C(=O)-O-$(CH_2)_7$-$CF_2CF_3$ |
| G2 | $CH_2$=CH-C(=O)-O-$(CH_2)_7$-$(CF_2)_3CF_3$ |

[Table 5]

| Compound | ΔG (kcal/mol) | Compound | ΔG (kcal/mol) | ΔG difference (kcal/mol) |
|---|---|---|---|---|
| A1* | -0.97 | A2 | -1.52 | 0.55 |
| B1* | -0.96 | B2 | -1.77 | 0.81 |
| C1* | -0.63 | C2 | -1.30 | 0.67 |
| D1* | -0.39 | D2 | -1.15 | 0.76 |
| E1 | -1.28 | E2 | -2.36 | 1.08 |
| F1* | -0.92 | F2 | -1.59 | 0.67 |
| G1* | -0.81 | G2 | -1.33 | 0.52 |

With reference to Table 5, the ΔG of the polymers of compounds A1 to G1 were respectively higher than the

$\Delta G$ of the polymers of compounds A2 to G2 having inverted structures corresponding thereto (see $\Delta G$ difference in Table 5). It is understood from the result that higher water-repellency can be obtained by having a fluoroalkylene group at a position closer to the acrylic structure than by having a fluoroalkyl group at an end of the compound. Further, in each of the polymers of the fluorine-containing compounds A1 to D1 and F1 to G1 of the disclosure, $\Delta G$ was -1 kcal/mol or more. Hence, it is understood that sufficiently high water-repellency is obtained. Furthermore, since the $\Delta G$ of the polymer of compound A1 and the $\Delta G$ of the polymer of compound B1 were substantially the same, it is understood that the presence or absence of an ether bond (-O-) does not substantially affect the resulting water-repellency. On the other hand, in each of polymers of compounds E1 and A2 to G2 of the comparative examples, $\Delta G$ was less than -1 kcal/mol, so that it is understood that the water-repellency was insufficient.

Industrial Applicability

The fluorine-containing compound of the disclosure can be used for various applications in which water-repellency is required. The fluorine-containing compound and/or the polymer containing a repeating unit derived from the fluorine-containing compound of the disclosure (referred to as "fluorine-containing compound or the like" of the disclosure) can impart water-repellency to an article by forming a surface of the article. Further, the composition containing the fluorine-containing compound of the disclosure or the like can be used as a water repellent to be used for imparting water-repellency to textile products and the like.

This application claims priority based on Japanese Patent Application No. 2020-100920 filed in Japan on June 10, 2020, and all the contents thereof are incorporated herein by reference.

Reference Signs List

S1, S2, S3, S4 Step

**Claims**

1. A compound represented by a general formula (1):

$$L\text{-}(R^1\text{-}X\text{-}Rf\text{-}Y\text{-}R^2)_n \ldots \qquad (1)$$

wherein L represents an n-valent organic group,
n represents an integer of 1 or more,
Rf represents a linear or branched fluoroalkylene group containing 1 to 4 of at least any of a $CF_2$ unit, a CFH unit and a CFRf' unit, and optionally having an ether bond, wherein Rf' represents a fluoroalkyl group having 1 carbon atom,
$R^1$ represents a direct bond, or a linear or branched hydrocarbon group having 1 to 4 carbon atoms,
$R^2$ represents a linear or branched hydrocarbon group having 7 to 29 carbon atoms, containing no fluorine atoms, and optionally having an ether bond, and
X and Y each independently represent a direct bond or a divalent linking group.

2. The compound according to claim 1, wherein in the general formula (1), X and Y represent direct bonds, and Rf is a linear or branched fluoroalkylene group containing 1 to 2 of at least any of a $CF_2$ unit, a CFH unit and a CFRf' unit.

3. The compound according to claim 1, wherein in the general formula (1), X and Y each independently represent a divalent linking group selected from the group consisting of $-SO_2-$, $-SO_2NR^3-$, $-NR^3SO_2-$, -O-, -C(=O)-O-, - O-C(=O)-, -C(=O) $-N(-R^3)-$, $-N(-R^3)-C(=O)-$, $-N(-R^3)-C(=O)-N(-R^3)-$, $-O-C(=O)-N(-R^3)-$, $-N(-R^3)-C(=O)O-$, and $-C_6H_4-$, wherein $R^3$ represents a hydrogen atom or a hydrocarbon group having 1 to 4 carbon atoms.

4. The compound according to any one of claims 1 to 3, wherein in the general formula (1), L is represented by a general formula (L-1):

$$CH_2\text{=}CA\text{-}Z\text{-}^* \ldots \qquad (L\text{-}1)$$

wherein A represents a hydrogen atom, a hydrocarbon group having 1 to 4 carbon atoms, a fluorine atom, a chlorine atom, a bromine atom, or an iodine atom,
Z represents a divalent linking group, and

symbol * represents a bonding hand of L; and
n represents 1.

**5.** The compound according to claim 4, wherein in the general formula (L-1), Z represents a divalent linking group selected from the group consisting of -C(=O)O-* and -C(=O)$NR^3$-* wherein $R^3$ represents a hydrogen atom, or a hydrocarbon group having 1 to 4 carbon atoms.

**6.** The compound according to any one of claims 1 to 3, wherein in the general formula (1), L is a carbon-containing linker moiety prepared by a process including reacting

(a) at least one isocyanate group-containing compound selected from the group consisting of isocyanate, diisocyanate and polyisocyanate, and
(b) at least one isocyanate reactive compound selected from the group consisting of compounds represented by general formulae (2a), (2b) and (2c):

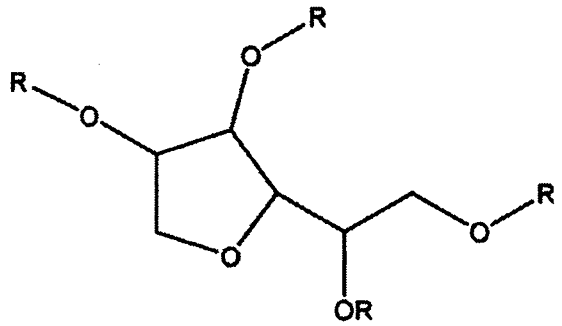

··· (2a)

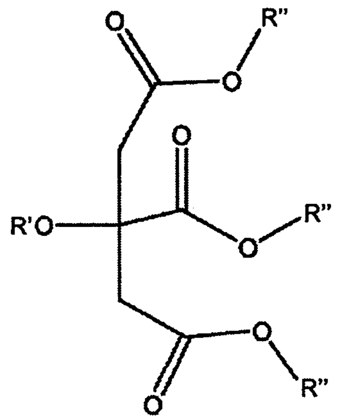

··· (2b)

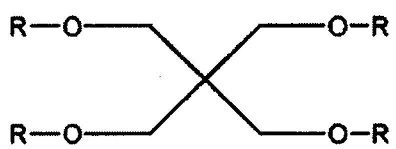

··· (2c)

wherein, in the general formulae (2a) and (2c),
each R independently represents -H, -*, -C(O)-*, - $(CH_2CH_2O)_p(CH(CH_3)CH_2O)_qH$, $-(CH_2CH_2O)_p(CH(CH_3)CH_2O)_q$-* , or - $(CH_2CH_2O)_p(CH(CH_3)CH_2O)_qC(O)$-*,
each p independently represents 0 to 20,
each q independently represents 0 to 20,
p+q is larger than 0,
symbol * represents a bonding hand of L, and
at least one R represents -H or - $(CH_2CH_2O)_p(CH(CH_3)CH_2O)_qH$, and at least another one R represents -*, -C(O)-*, $-(CH_2CH_2O)_p(CH(CH_3)CH_2O)_q$-*, or - $(CH_2CH_2O)_p(CH(CH_3)CH_2O)_qC(O)$-*, with the proviso that the isocyanate reactive compound is represented by the general formula (2a) or (2c) ; and

wherein, in the general formula (2b),
each R' independently represents -H, -*, -C(O)-*, - $(CH_2CH_2O)_{p'}(CH(CH_3)CH_2O)_{q'}H$, $-(CH_2CH_2O)_{p'}(CH(CH_3)CH_2O)_{q'}$-*, or $-(CH_2CH_2O)_{p'}(CH(CH_3)CH_2O)_{q'}C(O)$-*,
each R" independently represents -H, -*, - $(CH_2CH_2O)_{p'}(CH(CH_3)CH_2O)_{q'}H$, $-(CH_2CH_2O)_{p'}(CH(CH_3)CH_2O)_{q'}$-*, or $-(CH_2CH_2O)_{p'}(CH(CH_3)CH_2O)_{q'}C(O)$-*,
each p' independently represents 0 to 20,
each q' independently represents 0 to 20,
p'+q' is larger than 0,
symbol * is a bonding hand of L, and
at least one selected from R' and R" represents -H or $-(CH_2CH_2O)_{p'}(CH(CH_3)CH_2O)_{q'}H$, and at least another one selected from R' and R" represents -*, -C(O)-*, - $(CH_2CH_2O)_{p'}(CH(CH_3)CH_2O)_{q'}$-*, or - $(CH_2CH_2O)_{p'}(CH(CH_3)CH_2O)_{q'}C(O)$-*, with the proviso that the isocyanate reactive compound is represented by the general formula (2b).

7. The compound according to claim 6, wherein the diisocyanate and the polyisocyanate are at least one selected from the group consisting of hexamethylene diisocyanate homopolymer, 3-isocyanatemethyl-3,4,4-trimethylcyclohexyl isocyanate, bis-(4-isocyanatocyclohexyl)methane, and compounds represented by general formulae (3a), (3b), (3c) and (3d).

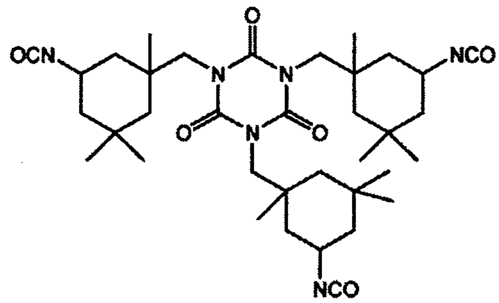

··· (3a)

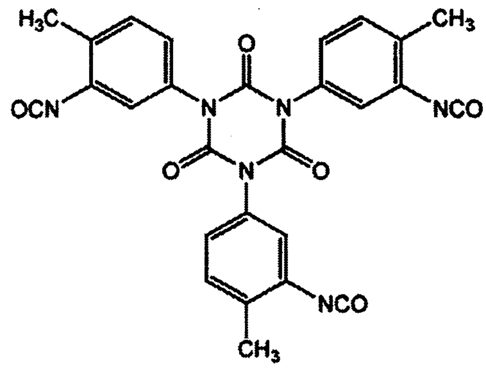

··· (3b)

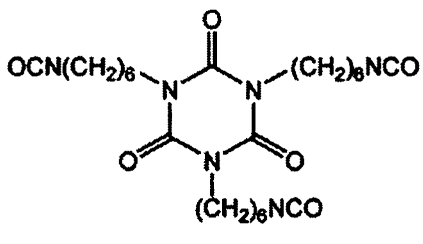

··· (3c)

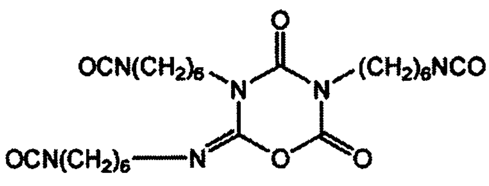

··· (3d)

**8.** A polymer, comprising a repeating unit derived from the compound according to claim 4 or 5.

**9.** An article having a surface formed of at least one selected from the group consisting of the compound according to any one of claims 1 to 7 and the polymer according to claim 8.

**10.** A composition, comprising:

at least one selected from the group consisting of the compound according to any one of claims 1 to 7 and the polymer according to claim 8; and
a liquid medium.

**11.** The composition according to claim 10, wherein the liquid medium is water or a mixture of water and an organic solvent.

**12.** The composition according to claim 10 or 11, which is a water repellent.

**13.** A textile product, which is processed with the composition according to any one of claims 10 to 12.

Fig. 1

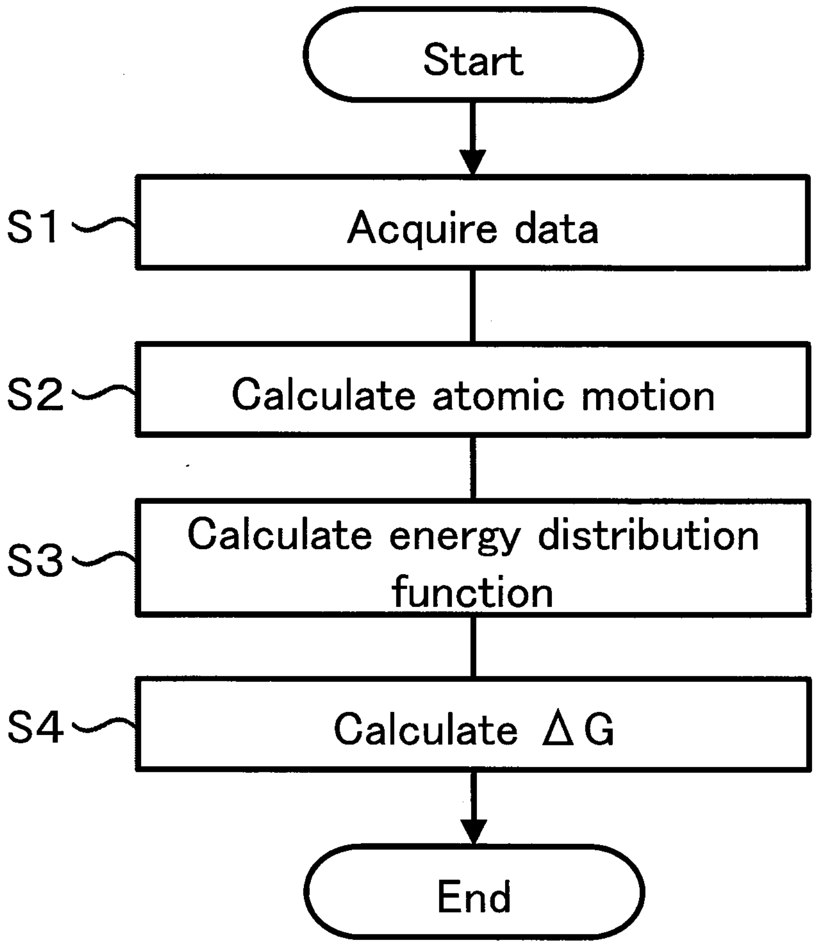
Start
S1
Acquire data
S2
Calculate atomic motion
S3
Calculate energy distribution function
S4
Calculate ΔG
End

Fig. 2

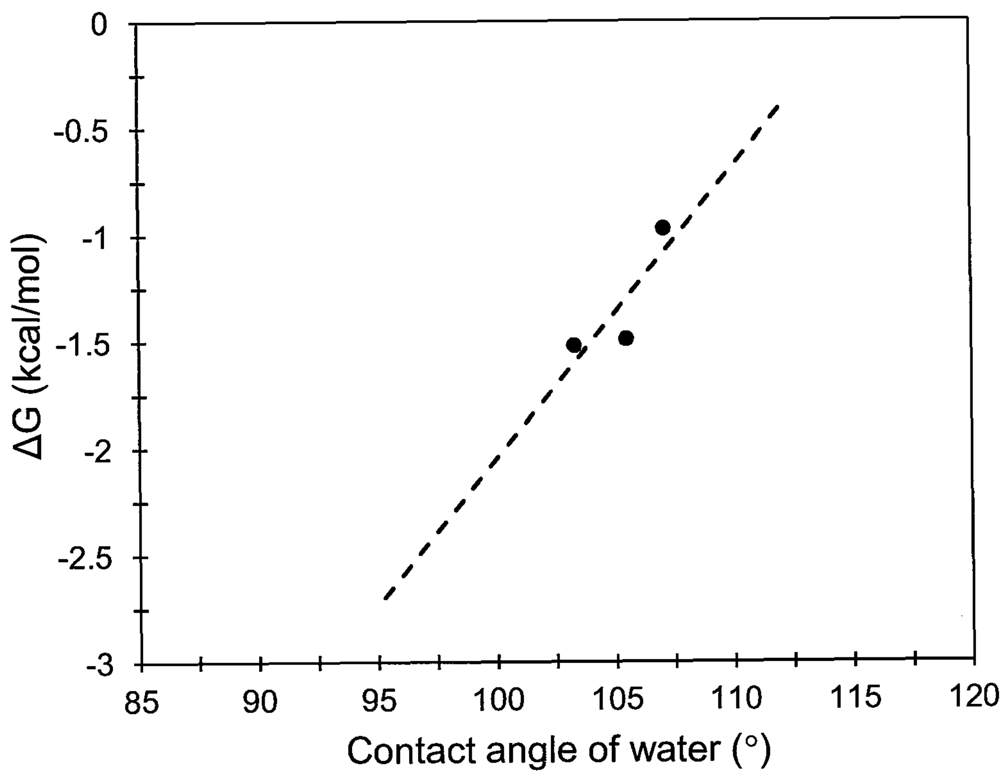
0
-0.5
-1
-1.5
-2
-2.5
-3
ΔG (kcal/mol)
85
90
95
100
105
110
115
120
Contact angle of water (°)

**INTERNATIONAL SEARCH REPORT**

International application No.
PCT/JP2021/021425

A. CLASSIFICATION OF SUBJECT MATTER
C08F 20/28(2006.01)i; C08F 20/58(2006.01)i; C09K 3/18(2006.01)i; C07C 69/653(2006.01)i
FI: C07C69/653 CSP; C09K3/18 102; C08F20/28; C08F20/58

According to International Patent Classification (IPC) or to both national classification and IPC

B. FIELDS SEARCHED

Minimum documentation searched (classification system followed by classification symbols)
C08F20/28; C08F20/58; C09K3/18; C07C69/653

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| X<br>Y<br>A | CASTELVETRO, V. et al., "Adapting the properties of new fluorinated acrylic polymers to suit the conservation of ancient monuments", Surface Coatings International, 1998, vol. 81, no. 11, pp. 551-556 page 552, column "Synthesis", pp. 554-555, column "Preliminary evaluation of protection efficiency on stones" | 1-2, 4-5, 8-12<br>13<br>3, 6-7 |
| Y<br>A | JP 2003-193370 A (DAIKIN INDUSTRIES, LTD.) 09 July 2003 (2003-07-09) claims | 13<br>1-12 |
| Y<br>A | WO 2013/058335 A1 (DAIKIN INDUSTRIES, LTD.) 25 April 2013 (2013-04-25) claims, paragraph [0049] | 13<br>1-12 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 09 July 2021 (09.07.2021) | 27 July 2021 (27.07.2021) |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No.
PCT/JP2021/021425

C (Continuation). DOCUMENTS CONSIDERED TO BE RELEVANT

| Category* | Citation of document, with indication, where appropriate, of the relevant passages | Relevant to claim No. |
|---|---|---|
| X<br>A | SCHOLZ, T. H. et al., "Sulfonylmethanesulfonamide inhibitors of carbonic anhydrase", Journal of Medicinal Chemistry, 1993, vol. 36, no. 15, pp. 2134-2141, Scheme III, Table I, page 2139, left column, line 12 from the bottom to right column, line 16 | 1, 3<br>2, 4-13 |
| X<br>A | HAGOOLY, Y. et al., "Constructing the OCF2O Moiety Using BrF3", Journal of Organic Chemistry, 2008, vol. 73, no. 17, pp. 6780-6783 scheme 2 | 1, 3<br>2, 4-13 |
| X<br>A | JP 2015-193615 A (TOYOTA CENTRAL R&D LABS., INC.) 05 November 2015 (2015-11-05) example 5 | 1, 3<br>2, 4-13 |
| X<br>A | JP 2016-102070 A (JNC CORPORATION) 02 June 2016 (2016-06-02) paragraph [0183] | 1, 3<br>2, 4-13 |

Form PCT/ISA/210 (continuation of second sheet) (January 2015)

**INTERNATIONAL SEARCH REPORT**
Information on patent family members

International application No.
PCT/JP2021/021425

| Patent Documents referred in the Report | Publication Date | Patent Family | Publication Date |
|---|---|---|---|
| JP 2003-193370 A | 09 Jul. 2003 | US 2003/0157256 A1<br>claims | |
| WO 2013/058335 A1 | 25 Apr. 2013 | TW 201326216 A | |
| JP 2015-193615 A | 05 Nov. 2015 | (Family: none) | |
| JP 2016-102070 A | 02 Jun. 2016 | US 2016/0152636 A1<br>p. 49 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2000290640 A
- JP 2020100920 A


### Non-patent literature cited in the description


- **S. SAKURABA et al.** ERmod: Fast and Versatile Computation Software for Solvation Free Energy with Approximate Theory of Solutions. *Journal of Computational Chemistry,* 2014, vol. 35 (21), 1592-1608